# EUROPÄISCHE PATENTSCHRIFT

(11) **EP 0 558 974 B1**
(45) Veröffentlichungstag und Bekanntmachung des Hinweises auf die Patenterteilung: **31.01.1996**
(21) Anmeldenummer: 93102189.3
(22) Anmeldetag: 12.02.1993
(51) Int. Cl.: C11C 1/00

(54) **Verfahren zum Gewinnen ungesättigter Fettsäuren**
Process for manufacturing unsaturated fatty acids
Procédé pour la fabrication d'acides gras insaturés

(30) Priorität: 02.03.1992 DE 4206539
(43) Veröffentlichungstag der Anmeldung: 08.09.1993
(73) Patentinhaber: K.D. PHARMA GmbH, D-66450 Bexbach (DE)
(72) Erfinder: Engelhardt, Heinz, Prof. Dr., D-6601 Bübigen (DE); Krumbholz, Rudolf, Dr., D-6689 Merchweiler (DE); Lembke, Peter, D-6601 Eschringen (DE)
(74) Vertreter: Bernhardt, Winfrid, Dr.-Ing.

(56) Entgegenhaltungen:
- EP-A- 0 240 013
- US-A- 4 063 911
- US-A- 4 909 935
- PATENT ABSTRACTS OF JAPAN vol. 012, no. 358 26. September 1988
- PATENT ABSTRACTS OF JAPAN vol. 013, no. 592 26. Dezember 1989
- PATENT ABSTRACTS OF JAPAN vol. 011, no. 163 26. Mai 1987
- PATENT ABSTRACTS OF JAPAN vol. 005, no. 194 10. Dezember 1981

## Beschreibung

Die Erfindung betrifft ein Verfahren zum Gewinnen mindestens einer ungesättigten Fettsäure mit sechzehn oder mehr Kohlenstoffatomen im Molekül und/oder mindestens einer Verbindung einer solchen Fettsäure aus einer, insbesondere pflanzlichen oder tierischen, Mischung von Fettsäuren und/oder Fettsäureverbindungen.

Diese Fettsäuren oder Fettsäureverbindungen werden bisher, hauptsächlich aus Fischöl, extrahiert. In der Regel liegen Triglyceride vor.

Das für die Extraktion verwendete Lösungsmittel, z.B. Benzol, Dichlormethan oder Acetonitril, hinterläßt einen unerwünschten, zum Teil giftigen Rückstand in der Fettsäure.

Aus der US-A-4,063,911 ist ein Verfahren zum Analysieren von Fettsäuren und Fettsäureverbindungen durch Gaschromatographie bekannt, für das als stationäre Phase eine spezielle flüssige Polymergruppe vorgeschlagen und mittels ihrer Strukturformel definiert ist. Diese stationäre Phase ist thermisch stabil und führt daher auch bei Anwendung hoher Temperatur von bis zu 275°C nicht zu Störungen infolge Zerfalls und Verdampfung. Sie kann daher zum Analysieren bei hoher Temperatur verdampfender Fettsäuren verwendet werden. Sie weist gute Trenneigenschaften auf.

Die JP-A-63112536 (Patent Abstracts of Japan) hat ein Verfahren zum Gewinnen von Linolensäure aus einer Mischung von Fettsäuren bzw. deren Estern zum Inhalt. Es ist vorgeschlagen, dafür die Chromatographie mit einer mobilen Phase aus einem überkritischen Fluid, wie Kohlendioxid, anzuwenden. Das Kohlendioxid ist preiswert und ungiftig. Es ist keine Vakuumdestillation als letzter Schritt erforderlich, das Erzeugnis wird nicht denaturiert.

Der Erfindung liegt die Aufgabe zugrunde, ein Verfahren zu finden, mit dem sich die eingangs genannten Fettsäuren und Fettsäureverbindungen aus einer Mischung gewinnen lassen, ohne mit einem Fremdstoff belastet zu werden.

Gemäß der Erfindung werden die Fettsäuren und/oder Fettsäureverbindungen der Mischung, unmittelbar oder nach einer Umsetzung, in einer mobilen Phase aus überkritischem oder flüssigem Kohlendioxid in einer Säule über eine stationäre Phase geführt, die auf einem Grundgerüst eine Belegung mit einer Phase aufweist, die an mindestens einer Gruppe ihres Moleküls bzw. ihrer Moleküle mindestens ein freies Elektronenpaar aufweist und/oder die in ihrem Molekül bzw. ihren Molekülen mindestens eine Mehrfachbindung aufweist, und die mindestens eine zu gewinnende Fettsäure und/oder Fettsäureverbindung wird nach dem Durchgang der, sofern vorhanden, gesättigten Fettsäure oder Fettsäureverbindung gleicher Kohlenstoffatomzahl durch die Säule für sich oder in einer angereicherten Mischung aufgefangen.

Das Kohlendioxid, in dem die Fettsäuren oder Fettsäureverbindungen bei diesem Verfahren gelöst werden, verflüchtigt sich aus dem gewonnenen Eluat. Die erhaltene Fettsäure oder Fettsäureverbindung ist rückstandsfrei.

Während die in der Analytik zur Trennung in einer Chromatographiesäule in der Regel angewandte Belegung mit einer Octadecyl-Phase die ein- bzw. mehrfach ungesättigten Fettsäurenim folgenden seien die Fettsäureverbindungen nicht immer gesondert erwähnt - vor der gesättigten Fettsäure gleicher Kohlenstoffatomzahl eluieren läßt und damit zu Überschneidungen insbesondere der ungesättigten und der gesättigten Fettsäuren mit achtzehn und zwanzig Kohlenstoffatomen führt, was keine Trennung erlaubt, hält die nach der Erfindung vorgesehene Belegung die ungesättigten Fettsäuren länger fest und eröffnet damit die Möglichkeit, sie zu trennen. Zwar kann noch die Ölsäure (18:1) mit der Eicosansäure (20:0) vollständig zusammenfallen; die Linolsäure (18:2), die Linolensäure (18:3), die Arachidonsäure (20:4), die Eicosapentaensäure (20:5), die Docosapentaensäure (22:5) und die Docosahexaensäure (22:6) folgen jedoch erst anschließend und nacheinander. Die in der vorstehenden Aufzählung übergangenen Säuren sind von geringerer Bedeutung.

Von besonderer Bedeutung sind aber die Linolsäure und die Linolensäure als "essentielle" Fettsäuren, d.h. solche, die der menschliche Körper nicht selbst aufbauen kann, sowie die Arachidonsäure, die Eicosapentaensäure und die Docosahexaensäure als "Omega-3-Fettsäuren", d.h. solche, die nach derzeitigem Kenntnisstand Fettablagerungen in Blutgefäßen aufzulösen imstande sind. Sie können zur Herstellung diätetischer oder pharmazeutischer Erzeugnisse dienen. Die übrigen Fettsäuren können teils-teils gleichfalls zur Herstellung diätetischer, aber auch sonstiger gesunder Nährmittel verwendet werden.

Die Wahl der Belegungs-Phase wird man nach ihrer Eignung für das jeweilige Ausgangsprodukt und die zu gewinnende Säure treffen. Durch Optimierung der Druck- und Temperaturbedingungen kann man das Verfahren dem jeweiligen Fall noch näher anpassen. Temperaturerhöhung zieht die Eluate der verschiedenen Fettsäuren auseinander und kann daher im Einzelfall zu einer gewünschten Trennung führen. Druckerhöhung schiebt die Eluate zusammen; sie kann zur Beschleunigung des Verfahrens dienen, wo das aufzufangende Eluat mehr zeitlichen Abstand zum vorangehenden und zum folgenden hat als nötig.

Die Erfindung nutzt die Wechselwirkung zwischen dem genannten freien Elektronenpaar bzw. den Π-Elektronen der MehrfachBindung in der Belegungs-Phase einerseits und den Π-Elektronen der Doppel-Bindungen in den ungesättigten Fettsäuren andererseits.

Die durch die "Π-Π-Wechselwirkung" zustandekommende Bindung ist an sich bekannt. In eine ähnliche Beziehung können freie Elektronenpaare mit den Π-Elektronen einer Doppel-Bindung treten. Infolge dieser Bindungen werden die ungesättigten Fettsäuren beim Durchgang durch die Säule länger festgehalten als die gesättigten Fettsäuren.

Bei der, wie erwähnt, zu analytischen Zwecken gebrauchten Belegung der stationären Phase mit langkettigen Molekülen werden demgegenüber die gesättigten Fettsäuren stärker festgehalten, deshalb, weil sie sich auf größerer Länge an die Moleküle der Belegungs-Phase anlegen können und daher in höherem Maße den hier wirksamen van der Waalschen Anziehungskräften usw. ausgesetzt sind als die gesättigten Fettsäuren, deren Molekülketten regelmäßig an der Doppelbindung geknickt sind und demgemäß nur auf umso kürzerer Länge festgehalten werden können, je mehr Doppelbindungen sie aufweisen.
Umgekehrt erhöht bei der erfindungsgemäßen Belegung die Zahl der Doppel-Bindungen der ungesättigten Fettsäuren deren Retardierung; die Möglichkeit für Bindungen ist größer.

Haltewirkung unter Anlegen der Molekülkette auf einem Abschnitt ihrer Länge ist jedoch nach der Erfindung gleichfalls vorgesehen. Sie soll die Fettsäuren mit verschiedener Kohlenstoffatomzahl voneinander trennen, die aufgrund unterschiedlicher Kettenlänge wiederum statistisch unterschiedliche Möglichkeiten zur Ausübung von Haltekräften bieten.

Beides, die Wirkung auf die Doppelbindung und die Wirkung auf die Kettenlänge, soll sich addieren.

Mit Rücksicht auf die Retention der Ketten auf ihrer Länge kann es zweckmäßig sein, wenn auch die Belegungs-Phase aus Kettenmolekülen gebildet ist, allerdings kürzeren. Moleküle mit zwei bis acht Kohlenstoffatomen erscheinen geeignet.

Ringmoleküle kommen jedoch ebenfalls in Betracht.

Zur Bildung der Belegungs-Phase mit einem freien Elektronenpaar kann ein eine Aminogruppe aufweisender Stoff verwendet werden, günstig ist eine Aminopropyl-Phase. Mehrere freie Elektronenpaare bieten Nitrogruppen.

Zur Bildung der Belegungs-Phase mit einer Mehrfach-Bindung kann ein eine Phenylgruppe und/oder eine Cyangruppe aufweisender Stoff verwendet werden. Beispielsweise wird mit einer Cyanopropyl-Phase belegt.

Als Beispiel für ein Ringmolekül zur Belegung sei Tetrachlorphthalimid genannt.

Gemischte Belegungen sind grundsätzlich möglich. Das Grundgerüst wird in der Regel aus Silikagel oder Aluminiumoxid bestehen.

Die Erfindung schließt ein, ggf. eine Mehrzahl in Mischung miteinander eluierender ungesättigter Fettsäuren mit sechzehn oder mehr Kohlenstoffatomen zu gewinnen. Vielfach wird eine Mischung der Fettsäuren ebenso verwendbar sein wie eine einzelne Säure. Dann lohnt sich keine Anstrengung, sie zu trennen.
Grundsätzlich wird es aber oft möglich sein, Koeluate zu trennen, indem man sie dem Verfahren noch einmal unterwirft unter Anwendung einer anderen Belegungs-Phase.

Praktische Bedeutung kann diese Möglichkeit für eine angereicherte Mischung einer zu gewinnenden ungesättigten Fettsäure erlangen, insbesondere einer Mischung mit einer unerwünschten gesättigten Fettsäure.

Näher in Betracht steht auch die Möglichkeit, die natürlich vorkommenden Triglyceride zunächst unverändert dem Verfahren zu unterwerfen, um die zu gewinnende(n) Fettsäure(n) anzureichern, die Triglyceride dann zu spalten und aus der nun vorliegenden Mischung die einzelne(n) Fettsäure(n) abzutrennen durch nochmalige Anwendung des Verfahrens, in diesem Falle ggf. auch mit derselben Belegungs-Phase.
Auch unmittelbare Verwendung der angereicherten Mischungen ist möglich, vor allem zu Ernährungszwecken.

Sonst wird man die Triglyceride von vorn herein spalten, etwa sauer oder alkalisch oder, vorzugsweise, durch Umesterung.

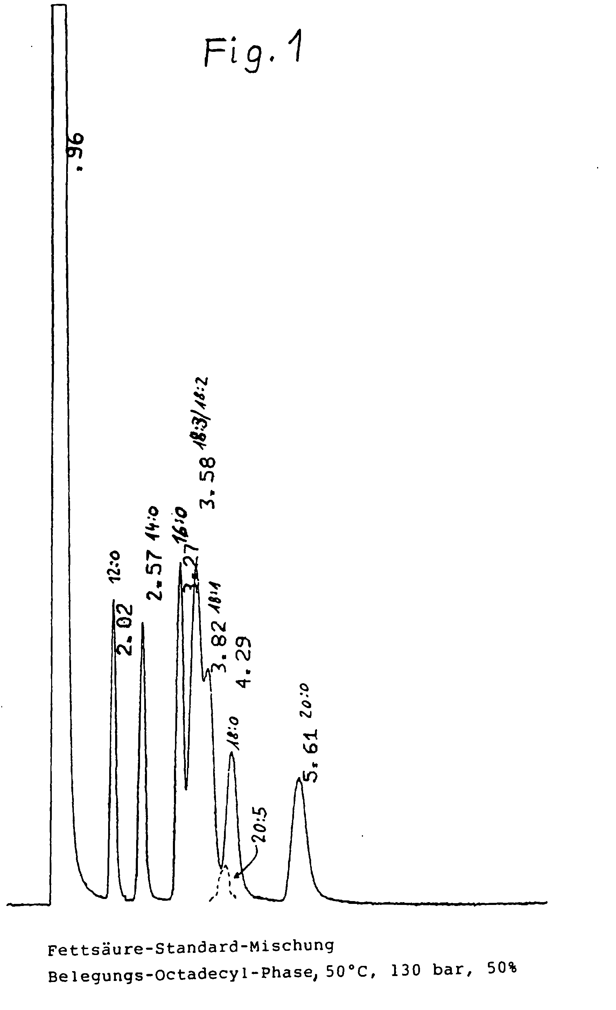

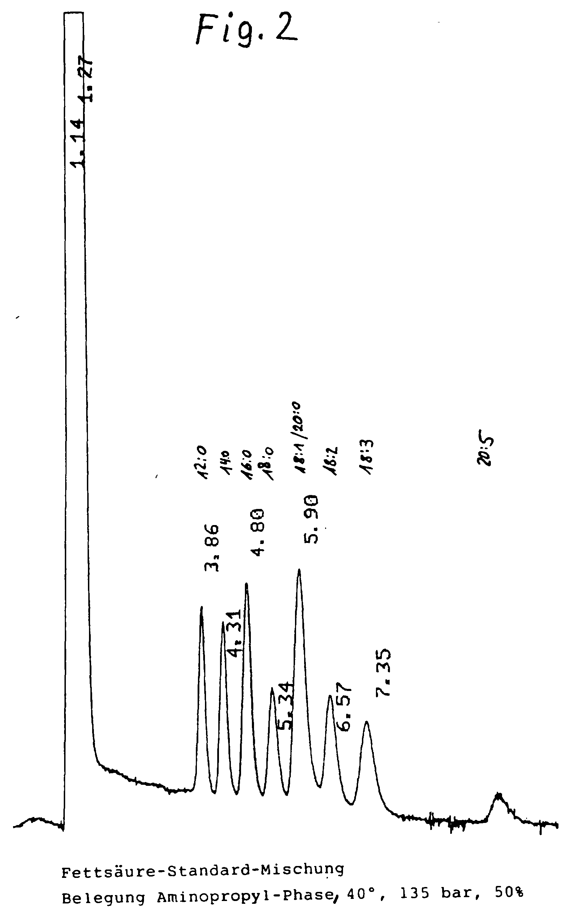

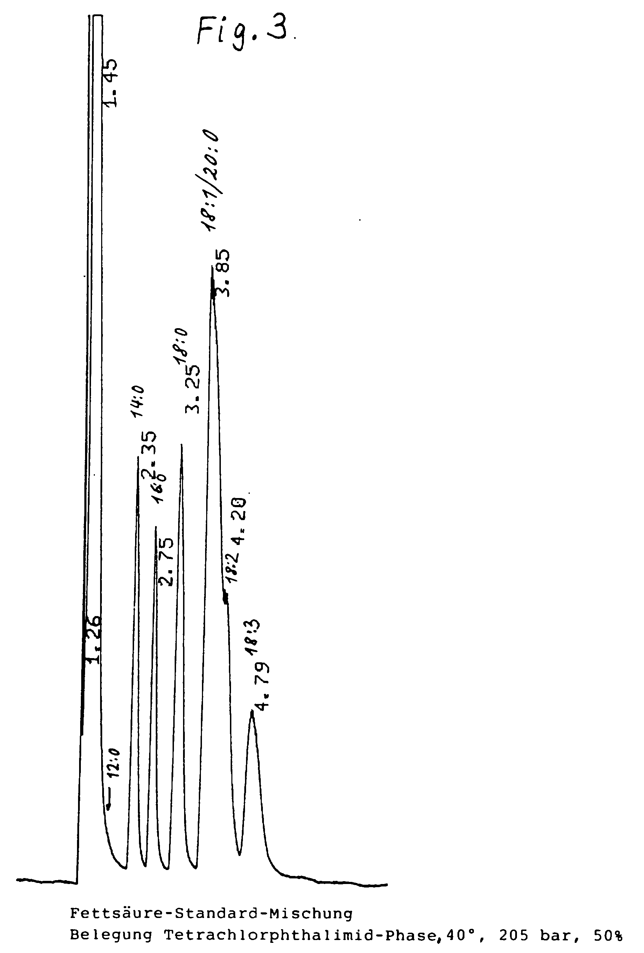

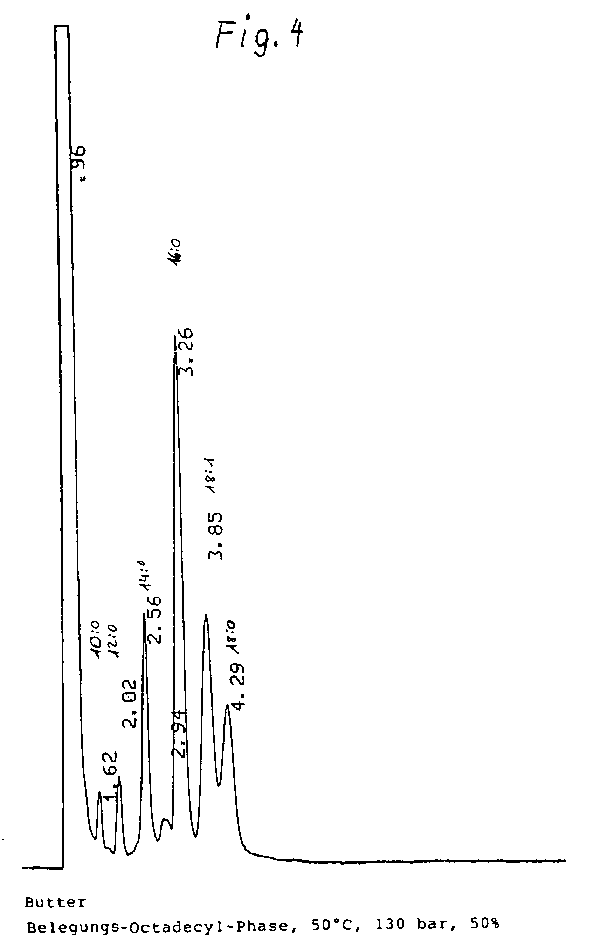

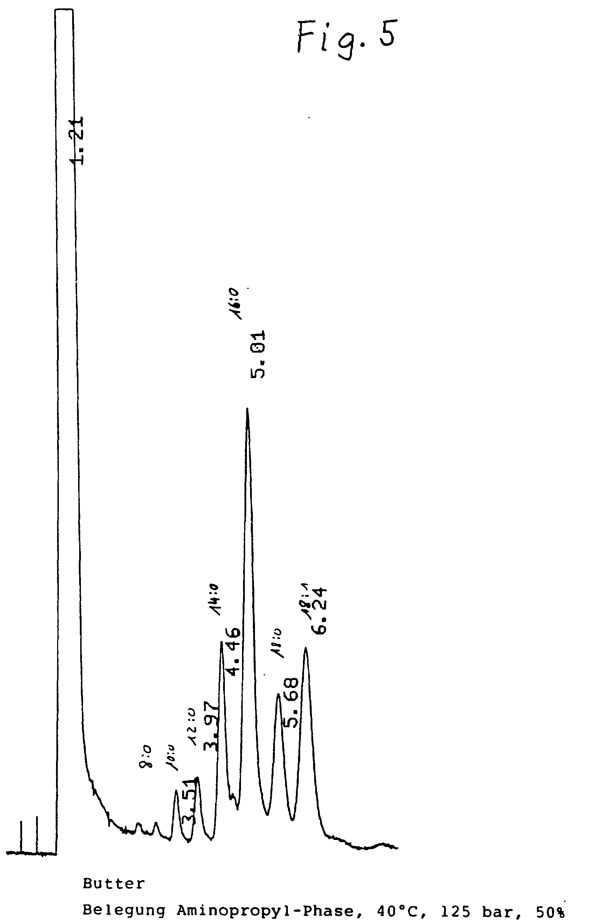

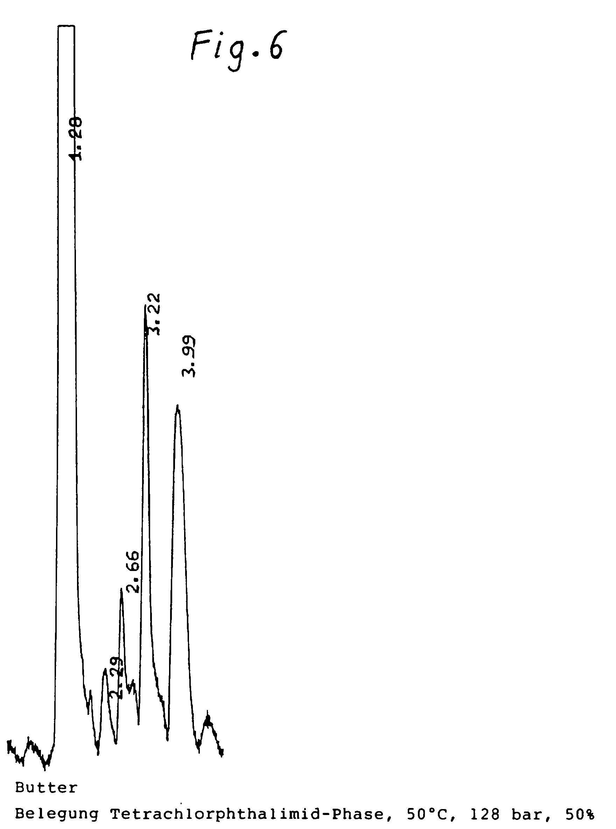

Im folgenden soll die Erfindung anhand von in Fig. 1 bis 11 dargestellten Versuchsergebnissen weiter erläutert werden.

Die Versuche wurden durchgeführt an einer Standardmischung sowie an verschiedenen natürlichen Fettsäuremischungen. Die Fettsäuren wurden in üblicher Verfahrenweise in Äthylester umgesetzt. Die Fettsäureestermischung wurde in Hexan gelöst, um sie schon als Lösung in überkritisches Kohlendioxid einzuführen, und mit diesem auf eine Säule von Silikagel mit in verschiedenen Versuchen verschiedenen Belegungs-Phasen gegeben. Der Säulenmantel bestand aus einem Edelstahlrohr von 25 cm Länge und 4 cm Innendurchmesser, das durch entsprechende weitere Anordnungen unter dem überkritischen Kohlendioxid gehalten wurde. Druck des Kohlendioxids und Temperatur wurden in verschiedenen Versuchen variiert. Die Eingabe der Probe erfolgte durch ein übliches Rheodyne-Ventil.

Die Eluate wurden als Chromatogramm erfaßt und aufgezeichnet.

Die Chromatogramme sind in Fig. 1 bis 11 wiedergegeben. Zur Erleichterung des Vergleichs sind darin jeweils das Ausgangsprodukt, die Belegungs-Phase, die Temperatur in °C, der Druck in bar und die Durchflußrate in % eingetragen. An den einzelnen peaks ist die Durchlaufzeit in Minuten (ohne Dimensionsangabe) vermerkt und/oder die betreffende Fettsäure in ihrer zahlenmäßigen Kurzform, siehe oben.

Das Versuchsergebnis nach Fig. 1 wurde aus einer Standard-Mischung von Fettsäuren mit einer Belegung des Silikagels mit der aus der Analysetechnik bekannten Octadecyl-Phase (C₁₈H₃₇) erhalten.

### Zusammensetzung der Mischung:

40 µg Laurinsäureäthylester (12:0)
41 µg Myristinsäureäthylester (14:0)
45 µg Palmitinsäureäthylester (16:0)
50 ug Linolsäureäthylester (18:2)
55 µg Linolensäureäthylester (18:3)
48 µg Ölsäureäthylester (18:1)
60 µg Stearinsäureäthylester (18:0)
46 µg Eicosansäureäthylester (20:0)

Der, hier nach 0,96 min gezeichnete, erste und größte peak ist derjenige des Lösungsmittels Hexan. Das gilt auch für alle weiteren Versuche.

Es folgen die gesättigten Fettsäuren 12:0, 14:0 und 16:0. Nach 16:0 und vor 18:0 eluieren zunächst ziemlich gleichzeitig 18:3 und 18:2 und dann in enger Überschneidung mit diesen 18:1. Als letztes folgt 20:0. Die ungesättigten Fettsäuren 20:4 und 20:5 und sogar 22:6 würden vor 20:0 erscheinen; gestrichelt eingetragen ist die besonders interessierende Eicosapentaensäure 20:5. Sie eluiert in Mischung hauptsächlich mit der Stearinsäure, aber auch der Ölsäure.

Fig. 2 zeigt die Verhältnisse nach Ersatz der Octadecyl-Phase durch Aminopropyl-Phase und Hinzufügung von Eicosapentaensäure zu der Standard-Mischung.
18:0 folgt unmittelbar auf 16:0. 18:1, 18:2 und 18:3 eluieren in dieser Reihenfolge nach 18:0, 18:1 dabei in Mischung mit 20:0. Die peaks der Linolsäure, der Linolensäure und der Eicosapentaensäure sind im wesentlichen bis zur Basis voneinander getrennt.

Fig. 3 zeigt wiederum für die Fig. 1 zugrunde liegende Standard-Mischung das Ergebnis mit Tetrachlorphthalimid-Phase als Belegung.
Auch hier eluieren 18:1, 18:2 und 18:3 nach 18:0. 18:3 kommt ziemlich isoliert. Mit geringerem Druck und höherer Temperatur könnte man die Eluate auseinanderziehen.

Fig. 4 bis 6 entsprechen etwa Fig. 1 bis 3, zeigen jedoch die Verhältnisse an einer natürlichen Fettsäuren-Mischung, nämlich aus der Butter. Diese Versuche sind lediglich von grundsätzlichem Interesse. Die Ölsäure könnte immerhin abgetrennt werden, wie Fig. 5 erkennen läßt.

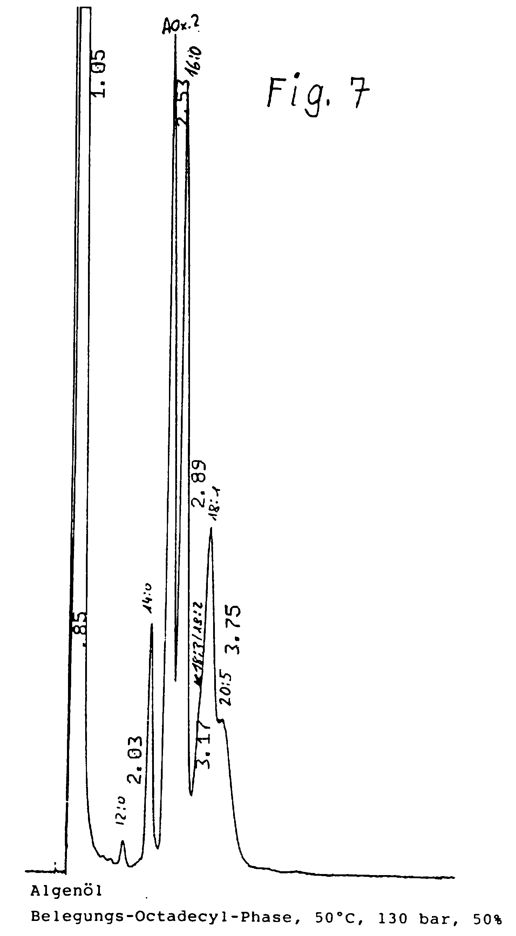

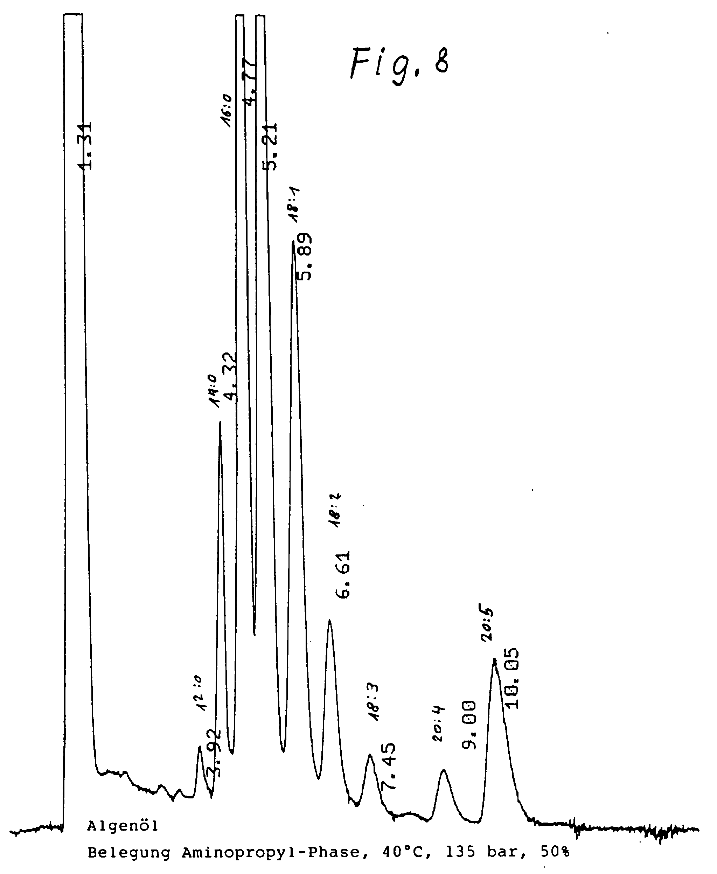

Das in den Versuchen gemäß Fig. 7 und 8 behandelte Algenöl kommt tatsächlich als Ausgangsprodukt für die Gewinnung von Eicosapentaensäure sowie Arachidonsäure, bei Optimierung möglicherweise auch von Linolsäure und Linolensäure, in Betracht. Der in Fig. 7 nur noch als Andeutung erscheinende peak der Eicosapentaensäure steht in Fig. 8 vollständig isoliert, desgleichen der in Fig. 7 ganz überdeckte peak der Arachidonsäure.

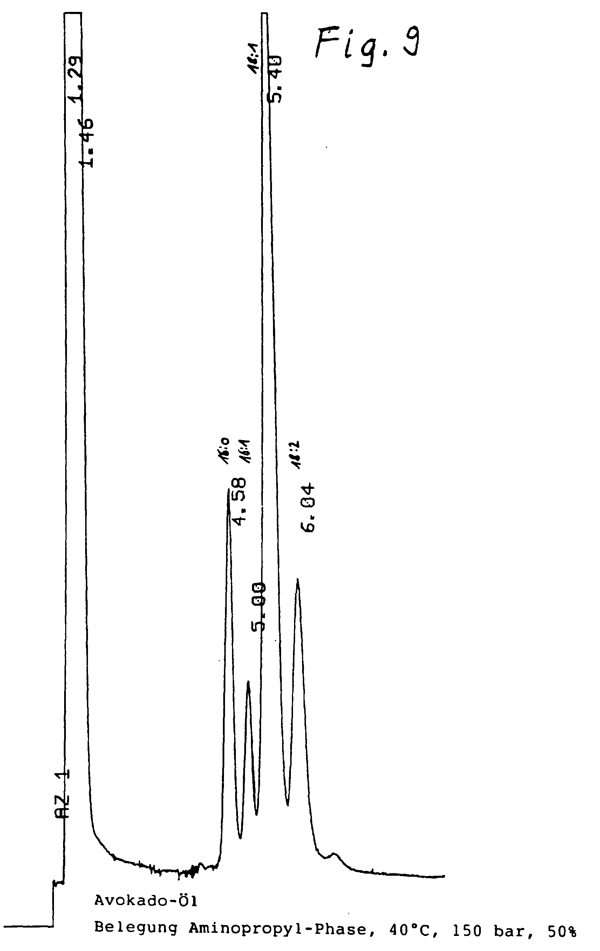

Fig. 9 läßt erkennen, daß aus Avokado-Öl in hohem Maße Ölsäure gewonnen werden könnte, ferner Linolsäure. Die Behandlungsbedingungen können dafür optimiert werden; durch Temperaturerhöhung und Druckerniedrigung lassen sich die Eluate auseinanderziehen.

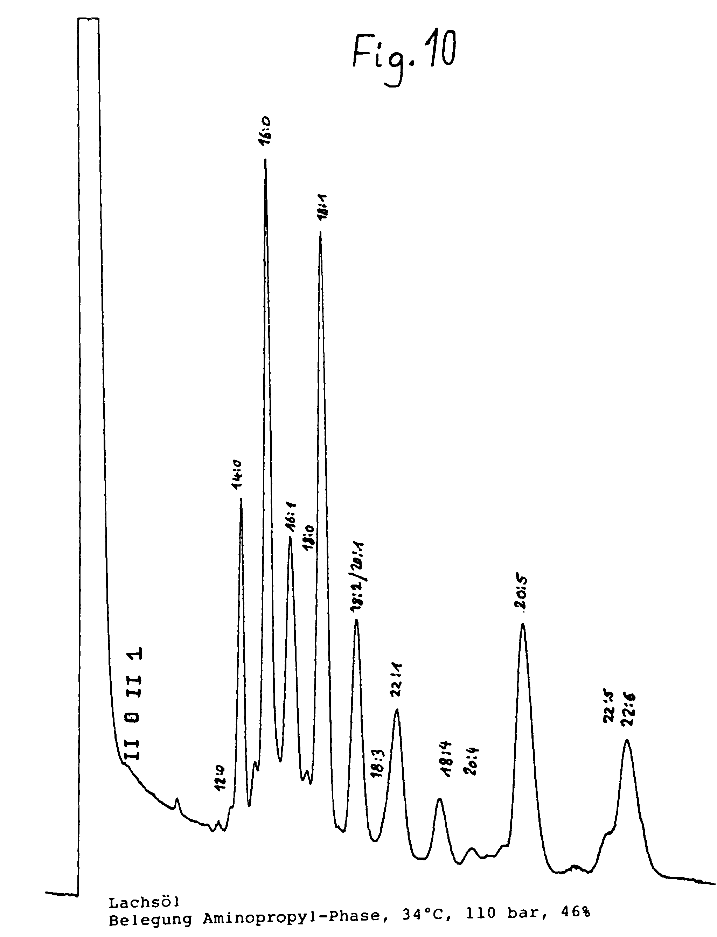

Aus Fig. 10 geht die Eignung von Lachsöl als Ausgangsprodukt für die Gewinnung von Eicosapentaensäure, Docosaensäure 22:1, einer Mischung von Docosahexaensäure 22:6 mit Docosapentaensäure 22:5, einer Mischung von Linolensäure 18:2 mit Eicosaensäure 20:1 sowie in geringerem Maße weiteren ungesättigten Fettsäuren hervor. Ausdrücklich erwähnt sei noch 16:1.

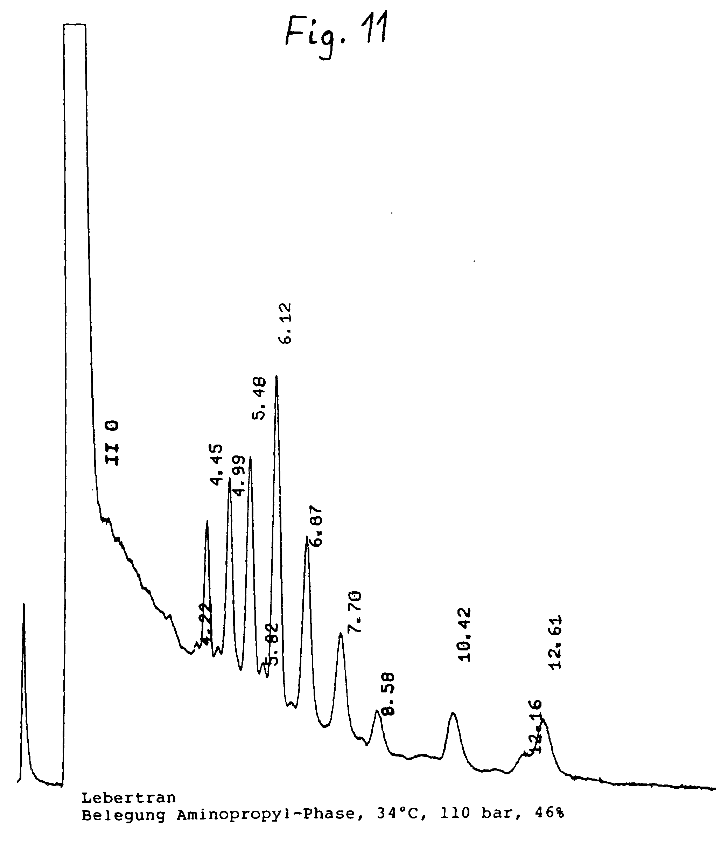

Auch aus Lebertran lassen sich ungesättigte Fettsäuren nach dem neuen Verfahren gewinnen. Das läßt die, wenn auch noch nicht ausgewertete, Fig. 11 pauschal erkennen.

## Patentansprüche

1. Verfahren zum Gewinnen mindestens einer ungesättigten Fettsäure mit sechzehn oder mehr Kohlenstoffatomen im Molekül und/oder mindestens einer Verbindung einer solchen Fettsäure aus einer, insbesondere pflanzlichen oder tierischen, Mischung von Fettsäuren und/oder Fettsäureverbindungen,
dadurch gekennzeichnet,
daß die Fettsäuren und/oder Fettsäureverbindungen der Mischung, unmittelbar oder nach einer Umsetzung, in einer mobilen Phase aus überkritischem oder flüssigem Kohlendioxid in einer Säule über eine stationäre Phase geführt werden, die auf einem Grundgerüst eine Belegung mit einer Phase aufweist, die an mindestens einer Gruppe ihres Moleküls bzw. ihrer Moleküle mindestens ein freies Elektronenpaar aufweist und/oder die in ihrem Molekül bzw. ihren Molekülen mindestens eine Mehrfachbindung aufweist, und daß die mindestens eine zu gewinnende Fettsäure und/oder Fettsäureverbindung nach dem Durchgang der, sofern vorhanden, gesättigten Fettsäure oder Fettsäureverbindung gleicher Kohlenstoffatomzahl durch die Säule für sich oder in einer angereicherten Mischung aufgefangen wird.

2. Verfahren nach Anspruch 1,
dadurch gekennzeichnet,
daß die Fettsäuren und/oder Fettsäureverbindungen in Äthylester umgesetzt werden.

3. Verfahren nach Anspruch 1 oder 2,
dadurch gekennzeichnet,
daß die genannte Mischung bzw. deren Umsetzungsprodukte vor ihrer Einführung in die mobile Phase in eine Lösung, vorzugsweise in Hexan, überführt wird.

4. Verfahren nach einem der Ansprüche 1 bis 3,
dadurch gekennzeichnet,
daß zur Bildung der Belegungs-Phase mit mindestens einem freien Elektronenpaar an einer Gruppe ein eine Aminogruppe oder Nitrogruppe aufweisender Stoff verwendet wird.

5. Verfahren nach einem der Ansprüche 1 bis 4,
dadurch gekennzeichnet,
daß zur Bildung der Belegungs-Phase mit einer MehrfachBindung ein eine Phenylgruppe und/oder eine Cyangruppe aufweisender Stoff verwendet wird.

6. Verfahren nach einem der Ansprüche 1 bis 5,
dadurch gekennzeichnet,
daß zur Bildung der Belegungs-Phase ein aus Kettenmolekülen, vorzugsweise mit zwei bis acht Kohlenstoffatomen, bestehender Stoff verwendet wird.

7. Verfahren nach Anspruch 4,
dadurch gekennzeichnet,
daß als Belegung eine Aminopropyl-Phase und/oder Tetrachlorphthalimid verwendet wird.

8. Verfahren nach Anspruch 5,
dadurch gekennzeichnet,
daß als Belegung eine Cyanopropyl-Phase verwendet wird.

## Claims

1. A process for manufacturing at least one unsaturated fatty acid with sixteen or more carbon atoms in the molecule and/or at least one compound of such a fatty acid from a particularly vegetable or animal mixture of fatty acids and/or fatty acid compounds,
characterised in that
the fatty acids and/or fatty acid compounds of the mixture are passed, directly or following a conversion, in a moving phase of supercritical or liquid carbon dioxide in a column over a stationary phase coated with a phase on a support and having at least one free electron pair at at least one group of its molecule or molecules and/or having at least one multiple bond in its molecule or molecules, and that the at least one fatty acid and/or fatty acid compound to be manufactured is collected alone or in an enriched mixture after the passage through the column of the saturated fatty acid or fatty acid compound, if present, of the same carbon number.

2. The process according to Claim 1,
characterised in that
the fatty acids and/or fatty acid compounds are converted to ethyl ester.

3. The process according to Claim 1 or 2,
characterised in that
the given mixture and/or its conversion products is/are put into solution, preferably in hexane, prior to introduction into the moving phase.

4. The process according to one of Claims 1 to 3,
characterised in that
a substance having an amino group or a nitro group is used for the formation of the coating phase with at least one free electron pair at a group.

5. The process according to one of Claims 1 to 4,
characterised in that
a substance having a phenyl group and/or a cyano group is used for the formation of the coating phase with a multiple bond.

6. The process according to one of Claims 1 to 5,
characterised in that
a substance consisting of chain molecules, preferably with two to eight carbon atoms, is used for the formation of the coating phase.

7. The process according to Claim 4,
characterised in that
an aminopropyl phase and/or tetrachlorophthalimide is used for the coating.

8. The process according to Claim 5,
characterised in that
a cyanopropyl phase is used for the coating.

## Revendications

1. Procédé pour la production d'au moins un acide gras insaturé dont la molécule comporte 16 atomes de carbone ou plus et/ou au moins un composé d'un tel acide gras à partir d'un mélange, en particulier végétal ou animal, d'acides gras et/ou de composés d'acides gras, caractérisé en ce que les acides gras et/ou les composés d'acides gras du mélange, sont passés, directement ou après une réaction, dans une phase mobile de dioxyde de carbone surcritique ou liquide, dans une colonne sur une phase stationnaire présentant sur une structure de base un revêtement avec une phase qui présente sur au moins un groupe de sa molécule ou de ses molécules au moins une paire d'électrons libres et/ou dont la molécule ou les molécules présentent au moins une liaison multiple et en ce que l'au moins un acide gras et/ou composé d'acide gras à produire est récupéré tel quel ou dans un mélange enrichi après le passage à travers la colonne de l'acide gras ou du composé d'acide gras saturé présentant le même nombre d'atomes de carbone, pour autant que celui-ci est présent.

2. Procédé selon la revendication 1, caractérisé en ce que les acides gras et/ou les composés d'acides gras sont transformés en esters éthyliques.

3. Procédé selon la revendication 1 ou 2, caractérisé en ce que le mélange mentionné ou le mélange de ses produits de réaction est mis dans une solution, de préférence dans de l'hexane, avant son introduction dans la phase mobile.

4. Procédé selon une quelconque des revendications 1 à 3, caractérisé en ce qu'on utilise une substance présentant un groupe amino ou nitro pour la formation de la phase de revêtement comportant au moins une paire d'électrons libres sur un groupe.

5. Procédé selon une quelconque des revendications 1 à 4, caractérisé en ce qu'on utilise une substance présentant un groupe phényle et/ou un groupe cyano pour la formation de la phase de revêtement comportant une liaison multiple.

6. Procédé selon une quelconque des revendications 1 à 5, caractérisé en ce qu'on utilise une substance constituée par des molécules en forme de chaîne, comportant de préférence deux à huit atomes de carbone pour la formation de la phase de revêtement.

7. Procédé selon la revendication 4, caractérisé en ce qu'on utilise comme revêtement une phase aminopropyle et/ou tétrachlorophtalimide.

8. Procédé selon la revendication 5, caractérisé en ce qu'on utilise comme revêtement une phase cyanopropyle.
